# EUROPEAN PATENT APPLICATION

(11) **EP 4 663 130 A1**
(43) Date of publication of application: **17.12.2025**
(21) Application number: 25182190.6
(22) Date of filing: 11.06.2025
(51) Int. Cl.: A61B 6/10

(54) **EXAMINEE ARRANGING APPARATUS FOR X-RAY**

(30) Priority: 13.06.2024 KR 20240077204
(71) Applicant: Vatech Co., Ltd., Hwaseong-si, Gyeonggi-do 18449 (KR); Vatech Ewoo Holdings Co., Ltd., Hwaseong-si, Gyeonggi-do 18449 (KR)
(72) Inventor: KIM, Sung Min, 18449 Gyeonggi-do (KR)
(74) Representative: Lorenz & Kollegen

(57) **Abstract**

Proposed is an examinee arranging apparatus for an X-ray imaging device that automatically stops when the pressure applied to an examinee reaches a reference value. The apparatus includes a guide portion installed to be movable for arranging a posture of a subject by applying pressure to the subject by the movement, an operating driving portion for controlling the movement of the guide portion, an elasticity portion for causing elasticity deformation in response to a repulsive force applying to the guide portion, a switch for controlling the operating driving portion to stop the movement of the guide portion when the elasticity deformation reaches a reference value.

## Description

### CROSS REFERENCE TO RELATED APPLICATION

The present application claims priority to Korean Patent Application No. 10-2024-0077204, filed on June 13, 2024, the entire contents of which are incorporated herein for all purposes by this reference.

### BACKGROUND

### Technical Field

The present disclosure relates to an examinee arranging apparatus for an X-ray imaging device that automatically stops when the pressure applied to an examinee reaches a reference value.

### Description of the Related Art

In general, dental clinics are equipped with X-ray imaging devices for taking X-rays in order to determine the condition of teeth and alveolar bones for the purpose of treating teeth and various periodontal diseases or correcting teeth alignment.

The X-ray imaging device used in dentistry transmits a certain amount of X-rays to the head, the body part to be imaged, and obtains an X-ray image by detecting the intensity of the transmitted X-rays and converting the same into electrical signals corresponding to the intensity of the X-rays at each location.

The X-ray imaging device described above is also referred to as an extra-oral X-ray device, and includes a panoramic imaging device, a CT imaging device, and an imaging device equipped with a panoramic imaging function and a CT imaging function, which are equipped with an examinee arranging apparatus for arranging the head of the examinee to be photographed in the correct position and posture.

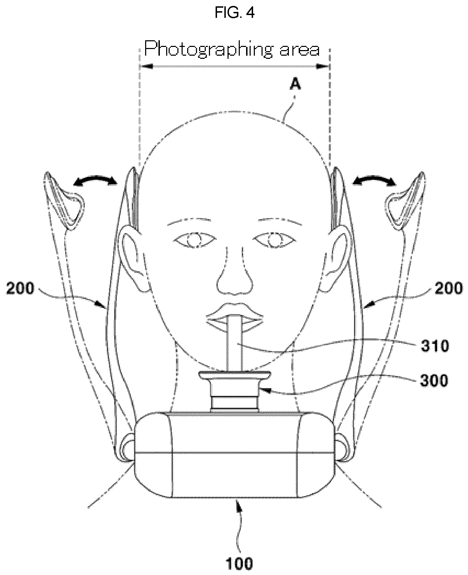

FIG. 4 is a front view showing a state where the head of an examinee is arranged through a conventional dental examinee arranging apparatus.

The examinee arranging apparatus for arranging the head of the examinee (A) into the photographing position in the dental X-ray imaging device includes a base portion 100, a temple supporter 200, and a chin rest portion 300, as shown in FIG. 4.

The base portion 100 is a part where the temple supporter 200 and the chin rest portion 300 are installed, and a manual rotation means (not shown) for rotating the temple supporter 200 in an arc shape can be embedded inside the base portion 100.

The conventional examinee arranging apparatus described above may apply excessive pressure to the examinee's head due to inexperienced manual operation and, in particular, as the temple supporter 200 moves along the arc shape, the pressure may be concentrated at the end as shown in FIG. 4, thereby causing pain.

### [Related Art Documents]

### [Patent Document]

(Patent Document 1) Korean Patent No. 10-1659177

### SUMMARY

The present disclosure is contrived to solve the problem described above and aims to provide an examinee arranging apparatus for an X-ray imaging device which can prevent unnecessary pain by enabling the examinee arranging apparatus to pressurize an examinee at an appropriate pressure.

In order to achieve the objective described above, an examinee arranging apparatus for an X-ray imaging device includes a guide portion installed to be movable for arranging a posture of a subject by applying pressure to the subject by the movement, an operating driving portion for controlling the movement of the guide portion, an elasticity portion for causing elasticity deformation in response to a repulsive force applying to the guide portion, a switch for controlling the operating driving portion to stop the movement of the guide portion when the elasticity deformation reaches a reference value.

There are following effects according to an examinee arranging apparatus for an X-ray imaging device of the present disclosure.

When the guide portion comes into contact with the examinee's head and presses further than a certain pressure, the operating driving portion is automatically stopped to prevent unnecessary pain.

In addition, the guide portions are arranged and moved parallel to each other, so that the pressure is not concentrated on the examinee's head.

### BRIEF DESCRIPTION OF THE DRAWINGS

FIGS. 1 to 3 are plan views showing an operation order of an examinee arranging apparatus of an X-ray imaging device according to a preferred exemplary embodiment of the present disclosure.
FIG. 4 is a front view showing a state where the head of an examinee is arranged through a conventional dental examinee arranging apparatus.

### DETAILED DESCRIPTION OF THE DISCLOSURE

Hereinafter, a preferred exemplary embodiment according to the present disclosure will be described in detail with reference to the attached drawings. The exemplary embodiments described in the present specification and the configurations illustrated in the drawings are only the most preferred exemplary embodiments of the present disclosure and do not represent all of the technical ideas of the present disclosure, so it should be understood that there are various equivalents and modified examples that can replace them at the time of this application.

In addition, the same reference numerals are assigned to the same parts as the conventional ones and detailed descriptions are omitted.

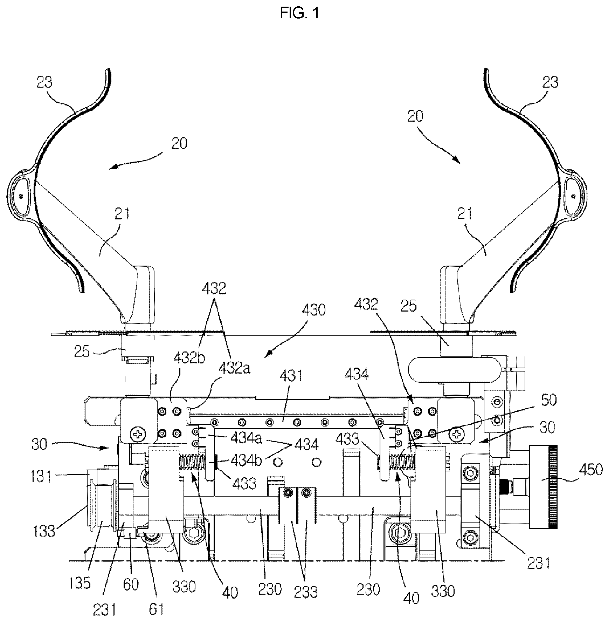

FIGS. 1 to 3 are plan views showing an operation order of an examinee arranging apparatus of an X-ray imaging device according to a preferred exemplary embodiment of the present disclosure.

Referring to FIGS. 1, 2, and 3, an X-ray imaging device according to the present exemplary embodiment may be, for example, a dental extra-oral X-ray imaging device, and an examinee arranging apparatus 1 may include a base 10, a guide portion 20 installed on the base 10 to be movable for arranging the posture of a subject by applying the pressure to the subject, which is the head of the examinee, an operating driving portion 30 for controlling a movement of the guide portion 20, an elasticity portion 40 for causing elasticity deformation in response to a repulsive force applying to the guide portion 20 from the subject by the movement of the guide portion 20, and a first switch 50 for controlling the operating driving portion 30 to stop the movement of the guide portion 20 when the elasticity deformation of the elasticity portion 40 reaches the reference value.

By such a configuration of the first switch 50, the pressure exceeding the elasticity of the elasticity portion 40 (e.g., the compression load of the compression coil spring is 500-510 gf) may not be applied to the examinee, thereby preventing pain from being caused by pressure.

The base 10 may include an examinee supporter (not shown). The examinee supporter may be a chin rest. The chin rest may be disposed between the support rods 25 to be described later. The base 10 may be supported on the main body (not shown). The examinee supporter (not shown) of the base 10 may be moved back and forth or left and right with respect to the main body (not shown) when viewed in a plan view.

The guide portion 20 may be, for example, a pair of temple supporters for pressing on the left and right temporal region of the examinee, and may be installed on the base 10 to be approachable/retractable from each other.

The guide portion 20 may include a pair of temporal pressing portions 21, a pressing side 23 installed at the front end of the temporal pressing portion 21, and a support rod 25 connected to the temporal pressing portion 21. The temporal pressing portion 21 may be arranged to be inclined and spread out to the left and right. The pressing side 23 may be shaped to press the front and rear of the temporal region of the examinee. The support rods 25 may be arranged parallel to each other in the forward and backward directions with respect to the left and right sliding directions of the movement portion 432 to be described later.

By placing the support rod 25 at a right angle to the approach and retraction directions, the concentrated pressure applied to the temporal region can be relieved, thereby preventing the pain of the examinee in advance.

The operating driving portion 30 may be an actuator installed inside the base 10, configured to enable the guide portions 20 to move toward or away from each other.

The operating driving portion 30 may include a motor (not shown), a lead screw 230 for receiving rotational power of the motor (not shown), a nut portion 330 fastened to the left and right of the lead screw 230, and an driving guide portion 430 for guiding the movement of the guide portion 20 with respect to the approach or retraction of the nut portion 330.

A driving pulley 131 may be installed on a rotating shaft of a motor (not shown). The driving pulley 131 may transmit rotational power through the belt 135 to the driven pulley 133 installed at one end of the lead screw 230 to be described later.

The lead screw 230 may form a left-right symmetrical male screw thread around its center along the rod and may be installed to enable bidirectional rotation.

Both left and right ends of the lead screw 230 may be supported by the bearing portion 231 installed on the base 10. The bearing portion 233 may be also installed at the center of the lead screw 230.

The driven pulley 133 may be installed at one end of the lead screw 230.

The nut portion 330 may be fastened so as to be approachable/retractable to the left and right according to the rotational direction of the lead screw 230.

The driving guide portion 430 may include a linear rail 431 installed in a horizontal direction to the left and right of the base 10, a movement portion 432 for moving in a straight line along the linear rail 431, a shaft 433 installed to enable the nut portion 330 to slide, and a connection side 434 for connecting the movement portion 432 and the shaft 433.

The movement portion 432 may be a linear block moving along a linear guide, on which the horizontal rod 25 may be installed.

The movement portion 432 may include a slider 432a for sliding along a linear rail 431, and a block body 432b that is fastened and fixed to the slider 432a.

The shaft 433 may be disposed horizontally and may guide the nut portion 330 in the approach/retract direction.

The connection side 434 can support one end of the shaft 433 with respect to the block body 432b of the movement portion 432. The connection side 434 may be a ' 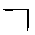 '-shape when viewed from the plane, and may include a first connection side 434a installed on one side of the block body 432b and a second connection side 434b which is extended from the first connection side 434a and is installed on one end of the shaft 433. A first switch 50 may be installed on the other side of the first connection side 434a.

By such a configuration of the operating driving portion 30, the elasticity portion 40 may be installed on an outer circumferential surface of any one of the left and right shafts 433. That is, the elasticity portion 40 may be disposed between the second connection side 434b and the nut portion 330. The elasticity portion 40 may be a coil compression spring. In the present exemplary embodiment, the elasticity portion 40 may be installed on the left and right shafts 433.

The operating driving portion 30 may further include a knob 450 exposed to one side of the base 10 for user operation, and a controller (not shown) for switching the rotation direction of the motor (not shown) to a forward or reverse direction according to the rotation direction of the knob 450.

That is, when a signal to tum the knob 450 to one side is transmitted to the controller (not shown), the motor (not shown) may be rotated in the forward direction to tighten the guide portion 20 and, conversely, when a signal to turn the knob 450 to the other side is transmitted to the controller (not shown), the motor (not shown) may be rotated in the reverse direction to loosen the guide portion 20, which is an automatic method.

The examinee arranging apparatus 1 for an X-ray imaging device according to an exemplary embodiment of the present disclosure may include a second switch 60 for stopping the operating driving portion 30 when the guide portion 20 reaches an initial position (see FIG. 1).

The second switch 60 may be installed on one of the left and right bearing portions 231. In the present exemplary embodiment, it may be installed on the underside of the bearing portion 231 on the left. At this time, a protrusion side 61 touching the second switch 60 may be formed on the nut portion 330 on the left side.

The first switch 50 and the second switch 60 may be implemented as limit switches or push switches.

Referring to FIGS. 1 to 3, the operation of the examinee arranging apparatus 1 for an X-ray imaging device having the configuration above may be described as follows.

As shown in FIG. 1, the state where the protrusion side 61 presses the second switch 60 and stops may be the initial state where the guide portions 20 are retracted from each other.

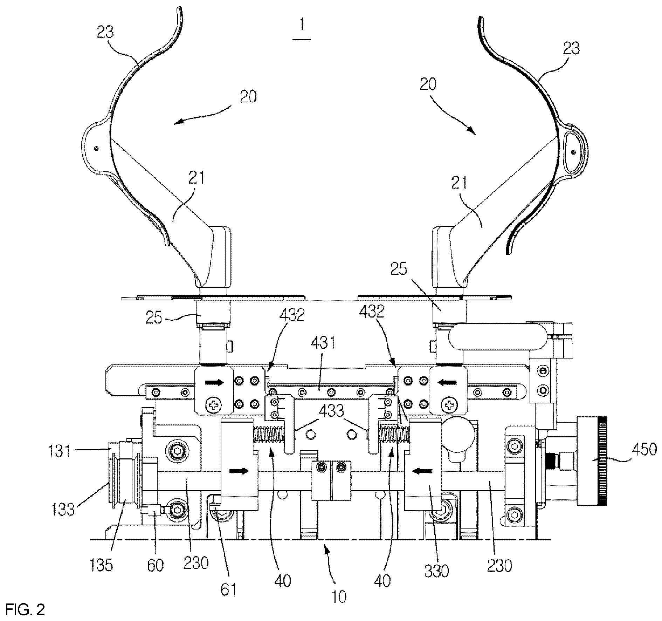

When the examinee puts the face between the retracted guide portions 20 and puts the chin on an examinee supporter (not shown) in a state where the guide portions 20 are retracted, the rotational power of the motor (not shown) may rotate the lead screw 230, thereby enabling the nut portions 330 to approach closer to each other in the direction of the arrow, as shown in FIG. 2.

The approach of the nut portion 330 may enable the driving guide portion 430 to approach together through the elasticity portion 40, so that the guide portion 20 can touch the temporal region of the examinee (see FIG. 2).

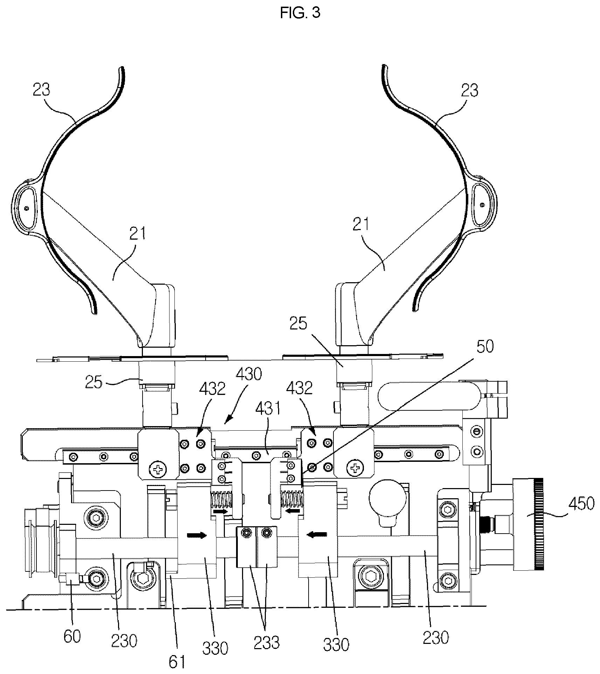

When the guide portion 20 touches the temporal region of the examinee as shown in FIG. 2, the nut portion 330 may continue to move in the approach direction and compress the elasticity portion 40, so that the guide portion 20 can gradually press the temporal region of the examinee as shown in FIG. 3.

When the guide portion 20 gradually presses the temporal region of the examinee, and the nut portion 330 presses the first switch 50 below the reference value of the compression load of the elasticity portion 40, the motor (not shown) may be stopped, so that the guide unit 20 may stop and align the temporal region of the examinee without pressing.

## Claims

1. An examinee arranging apparatus for an X-ray imaging device, the apparatus comprising:
a guide portion(20) installed to be movable for arranging a posture of a subject by applying pressure to the subject by the movement;
an operating driving portion(30) for controlling the movement of the guide portion(20);
an elasticity portion(40) for causing elasticity deformation in response to a repulsive force applying to the guide portion(20);
a switch for controlling the operating driving portion(30) to stop the movement of the guide portion(20) when the elasticity deformation reaches a reference value.

2. The apparatus of claim 1, wherein the elasticity portion(40) comprises a compression coil spring for causing compression elasticity deformation by the repulsive force.

3. The apparatus of claim 2, wherein the operating driving portion(30) comprises:
a motor;
a lead screw(230) rotating by the motor;
a nut portion(330) being fastened to the lead screw(230) and moving along the lead screw(230) by the rotation; and
a movement portion(432) connected to the guide portion(20) and movable together with the guide portion(20),
wherein the elasticity portion(40) is disposed between the nut portion(330) and the movement portion(432), and
the switch turns off the motor when a gap between the nut portion(330) and the movement portion(432) is less than or equal to a predetermined value.

4. The apparatus of claim 3, further comprising a linear rail(431) arranged parallel to the lead screw(230), wherein the movement portion(432) moves along the linear rail(431) while facing the nut portion(330).

5. The apparatus of claim 3, further comprising a shaft(433) installed in one of the nut portion(330) or the guide portion(20) and inserted into the other one of the nut portion(330) and the guide portion(20) through penetrating the elasticity portion(40).

6. The apparatus of claim 2, wherein compression load for the reference value of the compression elasticity deformation is 500 to 510 gf.

7. The apparatus of claim 3, wherein the operating driving portion(30) further comprising:
a knob(450) exposed to one side of a base for user operation; and
a controller for switching a rotation direction of the motor to a forward or reverse direction according to the rotation direction of the knob(450).

8. The apparatus of claim 6, wherein the switch is installed in one of the nut portion(330) and the movement portion(432), and is pressed by the other one of the nut portion(330)) and the movement portion(432) by the movement of the nut portion(330) and the movement portion(432), thereby turning off the motor.

9. The apparatus of claim 2, wherein the movement of the guide portion(20) stops when a length of the elasticity portion(40) reaches a certain value.
